(19) Europäisches Patentamt
European Patent Office
Office européen des brevets

(11) Veröffentlichungsnummer : **0 353 557 B1**

(12) # EUROPÄISCHE PATENTSCHRIFT

(45) Veröffentlichungstag der Patentschrift :
**16.09.92 Patentblatt 92/38**

(51) Int. Cl.$^5$ : **C07D 209/90,** A61K 31/40

(21) Anmeldenummer : **89113417.3**

(22) Anmeldetag : **21.07.89**

(54) **Tetrahydro-1-benz-[c,d]-indol-propionsäure-sulfonamide.**

(30) Priorität : **03.08.88 DE 3826371**

(43) Veröffentlichungstag der Anmeldung :
**07.02.90 Patentblatt 90/06**

(45) Bekanntmachung des Hinweises auf die Patenterteilung :
**16.09.92 Patentblatt 92/38**

(84) Benannte Vertragsstaaten :
**AT BE CH DE ES FR GB GR IT LI LU NL SE**

(56) Entgegenhaltungen :
**DE-A- 3 613 623**
**DE-A- 3 631 824**

(73) Patentinhaber : **BAYER AG**
**W-5090 Leverkusen 1 Bayerwerk (DE)**

(72) Erfinder : **Böshagen, Horst, Dr.**
**Wiesenstrasse 4**
**W-5657 Haan (DE)**
Erfinder : **Rosentreter, Ulrich, Dr.**
**Kondorweg 23**
**W-5600 Wuppertal 1 (DE)**
Erfinder : **Perzborn, Elisabeth, Dr.**
**Am Tescher Busch 13**
**W-5600 Wuppertal 11 (DE)**
Erfinder : **Fiedler, Volker-Bernd, Dr.**
**Lehner Mühle 46**
**W-5090 Leverkusen 3 (DE)**

**EP 0 353 557 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Tetrahydro-1-benz-[c,d]-indolpropionsäure-sulfonamide, Verfahren zu ihrer Herstellung und ihre Verwendung in Arzneimitteln.

Es ist bekannt, daß Cycloalkano-(1,2-b)-indol- und N-Dihydroindolylethyl-sulfonamide eine thrombozytenaggregationshemmende Wirkung aufweisen (DE-A 3 631 824 und 3 613 623).

Es wurden nun neue Tetrahydro-1-benz-[c,d]-indolpropionsäure-sulfonamide der allgemeinen Formel (I)

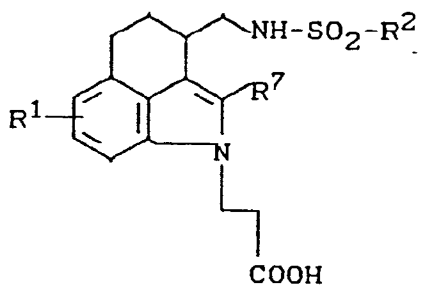

(I)

worin

$R^1$

– für Wasserstoff, Fluor, Chlor, Brom, Trifluormethyl, Carboxy oder $C_1$-$C_6$-Alkoxycarbonyl steht,

oder

– für eine Gruppe $-S(O)_nR^3$, $NR^4R^5$ oder $-OR^6$ steht,

worin

$R^3$ - $C_1$-$C_6$-Alkyl oder Phenyl bedeutet,

$R^4$ und $R^5$ gleich oder verschieden sind und Wasserstoff, $C_1$-$C_6$-Alkyl, Phenyl oder Benzyl bedeuten,

$R^6$ - Wasserstoff, $C_1$-$C_6$-Alkyl, Phenyl, Benzyl oder Trifluormethyl bedeutet,

und

n - eine Zahl 0 oder 2 bedeutet,

oder

$R^1$ - für $C_1$-$C_6$-Alkyl, $C_2$-$C_6$-Alkenyl, Cyclopropyl, Cyclopentyl oder Cyclohexyl steht, das gegebenenfalls durch Carboxy, $C_1$-$C_6$-Alkoxy, Fluor, Chlor, Brom, Hydroxy, $C_1$-$C_6$-Alkoxycarbonyl, $C_1$-$C_6$-Alkylthio oder Cyano substituiert ist,

$R^2$ - für Phenyl oder Naphthyl steht, das bis zu 3-fach gleich oder verschieden durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxycarbonylmethyl, Ethoxycarbonylmethyl, Methoxycarbonylethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio

oder durch eine Gruppe der Formel $-NR^4R^5$ substituiert ist,

worin

$R^4$ und $R^5$ die oben angegebene Bedeutung haben,

$R^7$ - für Wasserstoff, $C_1$-$C_6$-Alkyl, Cyclopropyl, Cyclopentyl oder Cyclohexyl steht,

und deren Salze

gefunden.

Die erfindungsgemäßen Verbindungen können in verschiedenen stereochemischen Formen existieren. Sowohl die einzelnen Isomere als auch deren Mischungen sind Gegenstand der Erfindung

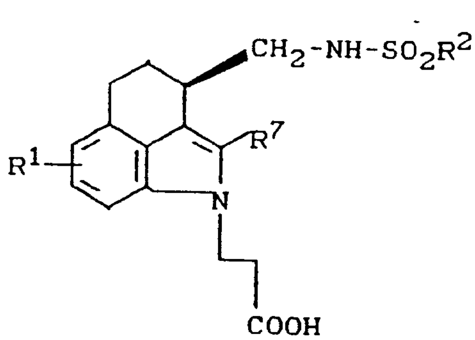

(II)

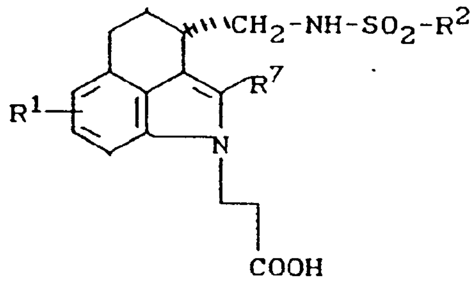

(III)

Die erfindungsgemäßen Verbindungen können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze können Metall- oder Ammoniumsalze der erfindungsgemäßen Stoffe, welche eine freie Carboxylgruppe besitzen, sein. Besonders bevorzugt sind z. B. Natrium-, Kalium-, Magnesium- oder Calciumsalze sowie Ammoniumsalze, die abgeleitet sind von Ammoniak oder organische Amine wie beispielsweise Ethylamin, Di- bzw. Triethylamin, Di- bzw. Triethanolamin, Dicyclohexylamin, Dimethylaminoethanol, Arginin oder Ethylendiamin.

Die erfindungsgemäßen Stoffe zeigen überraschenderweise eine thrombozytenaggregationshemmende Wirkung und können zur therapeutischen Behandlung von Menschen und Tieren verwendet werden.

Bevorzugt sind solche Verbindungen der allgemeinen Formel (I), in welcher

$R^1$ - für Wasserstoff, Fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino steht,

oder

– für eine Gruppe $-OR^6$ steht,

worin

$R^6$ - Wasserstoff, Methyl, Ethyl, Propyl, Phenyl oder Benzyl bedeutet

oder

– für Methyl, Ethyl, Propyl oder Isopropyl steht,

$R^2$ - für Phenyl oder Naphthyl steht, das bis zu 2fach gleich oder verschieden durch Fluor, Chlor, Brom, Cyano, Trifluormethyl, Trifluormethoxy, Methyl, Ethyl, Propyl, Methoxy, Ethoxy, Propoxy, Methylthio, Hydroxy, Methoxycarbonyl, Ethoxycarbonyl, Phenyl, Dimethylamino oder Diethylamino substituiert ist,

$R^7$ - für Wasserstoff oder Methyl steht,

und deren Salze.

Besonders bevorzugte Verbindungen der allgemeinen Formel (I) sind solche, in denen

$R^1$ - Wasserstoff oder Fluor bedeutet

$R^2$ - für Phenyl oder Naphthyl steht, das durch fluor, Chlor, Brom, Methyl, Methoxy oder Phenyl substituiert ist,

$R^7$ - für Methyl steht,

und deren Salze.

Beispielhaft seien folgende genannt:

2-Methyl-3-(benzolsulfonyl)-aminomethyl-1,3,4,5-tetrahydro-1-benz-[c,d]-indolpropionsäure

2-Methyl-3-(4-fluorbenzolsulfonyl)-aminomethyl-1,3,4,5-tetrahydro-1-benz-[c,d]-indolpropionsäure

2-Methyl-3-(4-brombenzolsulfonyl)-aminomethyl-1,3,4,5-tetrahydro-1-benz-[c,d]-indolpropionsäure

2-Methyl-3-(4-chlorbenzolsulfonyl)-aminomethyl-1,3,4,5-tetrahydro-1-benz-[c,d]-indolpropionsäure

2-Methyl-3-(4-toluolsulfonyl)-aminomethyl-1,3,4,5-tetrahydro-1-benz-[c,d]-indolpropionsäure

2-Methyl-3-(4-methoxybenzolsulfonyl)-aminomethyl-1,3,4,5-tetrahydro-1-benz-[c,d]-indolpropionsäure

2-Methyl-3-(1-naphthylsulfonyl)-aminomethyl-1,3,4,5-tetrahydro-1-benz-[c,d]-indolpropionsäure (Na-Salz).

Es wurde ein Verfahren zur Herstellung von Tetrahydro-1-benz-[c,d]-indolpropionsäure-sulfonamiden der allgemeinen Formel (I)

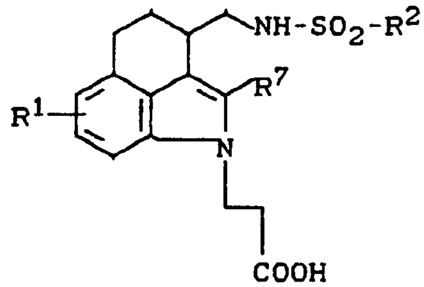

(I)

in welcher

$R^1$, $R^2$ und $R^7$ die oben angegebene Bedeutung haben,

gefunden, das dadurch gekennzeichnet ist, daß man Tetrahydro-1-benz-[c,d]-indol-sulfonamid-Verbindungen der allgemeinen Formel (IV)

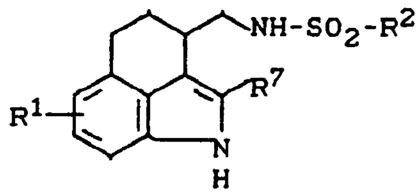

(IV)

in welcher

$R^1$, $R^2$ und $R^7$ die oben angegebene Bedeutung haben,

mit Acrylnitril der Formel (V)

$H_2C = CH\text{-}CN$ (V)

in Gegenwart eines inerten Lösemittels, gegebenenfalls in Gegenwart einer Base zu den Verbindungen der allgemeinen Formel (VI)

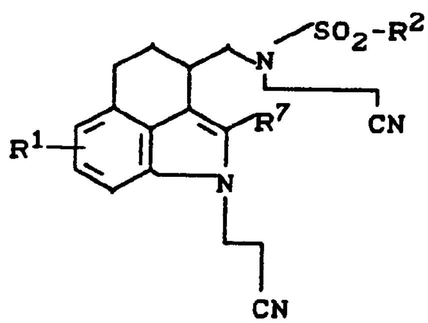

(VI)

in welcher

$R^1$, $R^2$ und $R^7$ die oben angegebene Bedeutung haben

umsetzt

und die N,N'-Biscyanoethylverbindungen (VI) anschließend verseift.

Das erfindungsgemäße Verfahren kann durch das folgende Formelschema erläutert werden:

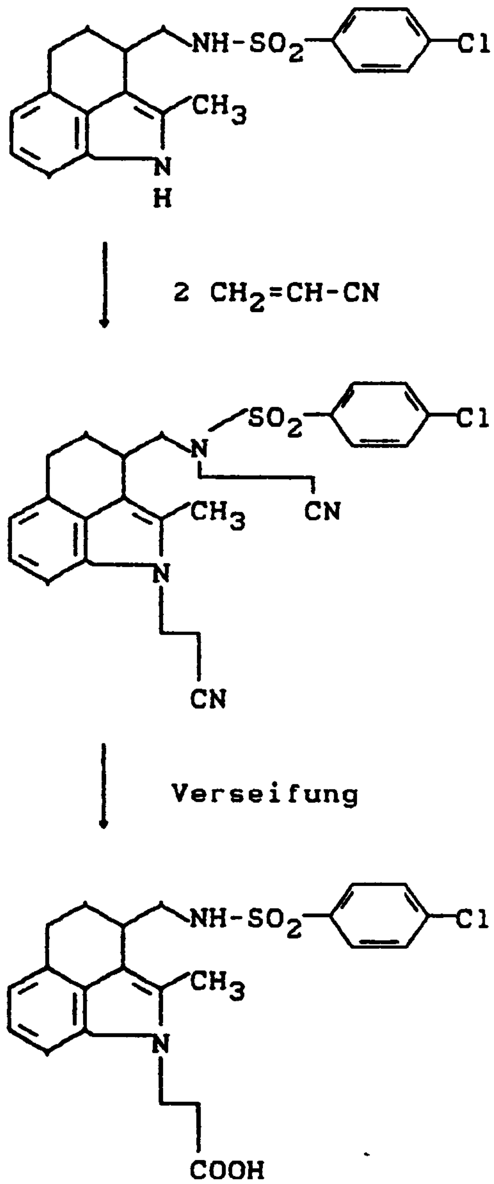


Lösungsmittel für das erfindungsgemäße Verfahren können inerte organische Lösungsmittel sein, die sich unter den Reaktionsbedingungen nicht verändern. Hierzu gehören bevorzugt Alkohole wie Methanol, Ethanol, Propanol oder Isopropanol, Ether wie Diethylether, Tetrahydrofuran, Dioxan, Glykolmono- oder -dimethylether, Kohlenwasserstoffe wie Benzol, Toluol, Xylol, Cyclohexan, Hexan oder Erdölfraktionen, Dimethylsulfoxid, Dimethylformamid, Hexamethylphosphorsäuretriamid, Essigester oder Pyridin. Ebenso ist es möglich, Gemische der genannten Lösungsmittel zu verwenden.

Basen für das erfindungsgemäße Verfahren können übliche basische Verbindungen sein. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat- oder -ethanolat oder Kalium-tert.-butylat, oder organische Amine wie Benzyltrimethylammoniumhydroxid, Tetrabutylammoniumhydroxid, Pyridin, Triethylamin oder N-Methylpiperidin.

Das erfindungsgemäße Verfahren wird im allgemeinen in einem Temperaturbereich von 0°C bis 150°C, bevorzugt von 20°C bis 100°C, durchgeführt.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen setzt man 2 bis 20 Mol, bevorzugt 2 bis 10 Mol Acrylnitril bezogen auf 1 Mol [Arylsulfo-

naminoalkyl]-1,3,4,5-tetrahydro-benz-[c,d]-indol ein.

Die Verseifung der N,N'-Bis-cyanoethylverbindungen erfolgt in an sich bekannter Weise in Gegenwart von Basen, wie Alkalioder Erdalkalihydroxiden oder -alkanolaten, in inerten Lösungsmitteln wie Wasser oder Alkoholen. Bevorzugt werden als Basen Natrium-, Kalium- oder Bariumhydroxid, Natriummethanolat, Kaliummethanolat, Natriumethanolat oder Kaliumethanolat, bevorzugt in Wasser oder Methanol, Ethanol, Propanol oder Isopropanol oder in Gemischen dieser Lösungsmittel eingesetzt.

Im allgemeinen setzt man 1 bis 100 Mol, bevorzugt 2 bis 50 Mol Base, bezogen auf 1 Mol N,N'-Biscyanoethylverbindung ein.

Die Verseifung wird in einem Temperaturbereich von 0°C bis 100°C, bevorzugt von 20°C bis 80°C durchgeführt.

Die Verbindungen der allgemeinen Formel (IV) sind neu. Es wurde ebenfalls ein Verfahren zur Herstellung gefunden, das dadurch gekennzeichnet ist, daß man Indole der allgemeinen Formel (VII)

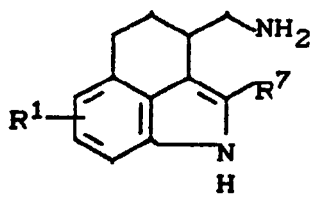

(VII)

in welcher

$R^1$ und $R^7$ - die oben angegebene Bedeutung haben

mit Sulfonsäurehalogeniden der allgemeinen Formel (VIII)

$$X\text{-}SO_2\text{-}R^2 \qquad (VIII)$$

in welcher

$R^2$ - die oben angegebene Bedeutung hat

und

X - für Halogen steht,

in inerten Lösemitteln, gegebenenfalls in Anwesenheit einer Base

umsetzt.

Die Reaktion kann durch folgendes Formelschema erläutert werden:

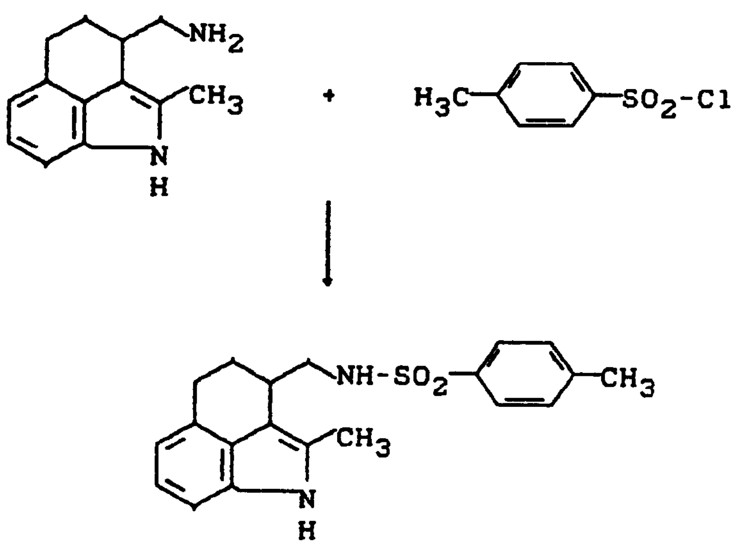

Lösungsmittel für das erfindungsgemäße Verfahren können hier inerte organische Lösungsmittel sein, die sich unter den Reaktionsbedingungen nicht verändern. Hierzu gehören bevorzugt Alkohole, wie beispielsweise Methanol, Ethanol, n-Propanol, iso-Propanol, Glykol, Ether wie beispielsweise Diethylether, Dioxan, Tetrahydrofuran, Glykolmono- oder -dimethylether, Halogenkohlenwasserstoffe wie Di-, Tri- oder Tetrachlormethan,

Dichlorethylen, Trichlorethylen, Essigester, Toluol, Acetonitril, Eisessig, Hexamethylphosphorsäuretriamid, Pyridin und Aceton. Selbstverständlich ist es möglich, Gemische der Lösungsmittel einzusetzen.

Basen für das erfindungsgemäße Verfahren können übliche basische Verbindungen sein. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat- oder -ethanolat oder Kalium-tert.-butylat, oder organische Amine wie Benzyltrimethylammoniumhydroxid, Tetrabutylammoniumhydroxid, Pyridin, Triethylamin oder N-Methylpiperidin.

Das erfindungsgemäße Verfahren wird im allgemeinen in einem Temperaturbereich von 0°C bis 150°C, bevorzugt bei 25 - 40°C, durchgeführt.

Das erfindungsgemäße Verfahren wird im allgemeinen bei Normaldruck durchgeführt. Es ist aber auch möglich, das Verfahren bei Unterdruck oder bei Überdruck durchzuführen (z. B. in einem Bereich von 0,5 bis 5 bar).

Im allgemeinen setzt man 1 bis 5 Mol, bevorzugt 1 bis 2 Mol, besonders bevorzugt 1 Mol Sulfonsäurehalogenid, bezogen auf 1 Mol des 3-(1-Aminoalkyl)-1,3,4,5-tetrahydrobenz-[c,d]-indols ein.

Als Sulfonsäurehalogenide können beispielsweise erfindungsgemäß verwendet werden:
Benzolsulfonylchlorid
4-Fluorbenzolsulfonylchlorid
4-Brombenzolsulfonylchlorid
4-Chlorbenzolsulfonylchlorid
4-Toluolsulfonylchlorid
4-Methoxybenzolsulfonylchlorid
1-Naphthylsulfonylchlorid

Die Verbindungen der allgemeinen Formel (VII) sind bekannt oder können nach bekannter Methode hergestellt werden (NL-A 6 409 079).

Die Sulfonsäurehalogenide der allgemeinen Formel (VIII) und Acrylnitril der Formel (V) sind bekannt [Houben-Weyls "Methoden der organischen Chemie", Band IX, S. 407 ff. und S. 547 ff. (1959)].

Die neuen Tetrahydro-1-benz-[c,d]-indolpropionsäure-sulfonamide bzw. deren Salze können als Wirkstoffe in Arzneimitteln eingesetzt werden. Die Wirkstoffe weisen eine thrombozytenaggregationshemmende und Thromboxan $A_2$-antagonistische Wirkung auf. Sie können bevorzugt zur Behandlung von Thrombosen, Thromboembolien, Ischämien, als Antiasthmatika und als Antiallergika eingesetzt werden. Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 5 bis 70 Gew.-%, bezogen auf die Zubereitung, vorliegen, die ausreichend ist, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nichttoxische organische Lösungsmittel wie Paraffine (z. B. Erdölfraktionen), pflanzliche Öle (z. B. Erdnuß/Sesamöl), Alkohole (z. B. Ethylalkohol, Glycerin), Glykole (z.B. Propylenglykol, Propylethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter

flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung im allgemeinen etwa 0,01 bis 20 mg/kg, vorzugsweise 0,1 bis 10 mg/kg Körpergewicht.

Es kann aber gegebenenfalls vorteilhaft sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen.

Die erfindungsgemäßen Tetrahydro-1-benz-[c,d]-indolpropionsäuresulfonamide können sowohl in der Humanmedizin als auch in der Veterinärmedizin angewendet werden.

Herstellungs- und Anwendungsbeispiele

Beispiel 1

2-Methyl-indol-3-succinsäure

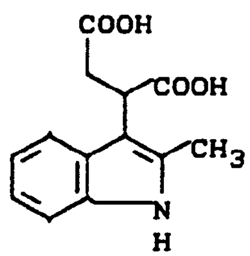

Eine Mischung aus 13,1 g (0,1 mol) 2-Methylindol und 11,6 g (0,1 mol) Maleinsäure werden solange auf einem Wasserbad erhitzt, bis sie sich verfestigt. Danach wird 30 Min. stehen gelassen. Anschließend wird eine Lösung aus 11,3 g (0,2 mol) Kaliumhydroxid in 200 ml Wasser hinzugefügt und die Mischung 30 Min. unter Rühren erhitzt. Nach dem Abkühlen wird die Lösung mit Ether extrahiert und mit Aktivkohle behandelt, filtriert, gekühlt und mit HCl angesäuert. Danach wird der Rückstand abfiltriert und mit Wasser gewaschen.

Fp.: 210-211° C (Zersetzung)

DC: $CHCl_3/CH_3OH$ = 3:1, Kieselgel auf Alufolie/Merck 5562

$R_f$ = 0,32

Ausbeute: 17,2 g (69,6 % d.Th.)

Beispiel 2

5-Fluor-2-methyl-indol-3-bernsteinsäure

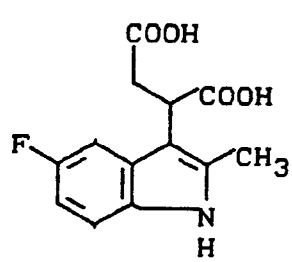

Die Titelverbindung wurde analog der Vorschrift von Beispiel 1 hergestellt.

Fp.: 220°C
DC : $CHCl_3/CH_3OH$ = 3 : 1, Kieselgel auf Alufolie/Merck 5562
$R_f$ = 0,21
Ausbeute: 57,1 %

Beispiel 3

1-Acetyl-2-methyl-3-indolsuccinanhydrid

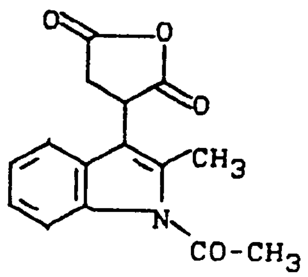

Eine Mischung aus 24,7 g (0,1 mol) 2-Methyl-3-indolsuccinsäure, 200 ml Isopropenylacetat und 2 g (0,011 mol) p-Toluolsulfonsäuremonohydrat wird 30 Min. unter Rückfluß erhitzt. Das Aceton wird durch Destillation langsam entfernt. Die Lösung wird im Vakuum eingeengt und der Rückstand aus 60 ml Essigsäure und 15 ml Essigsäureanhydrid kristallisert.
Fp.: 192-193°C
DC: $CHCl_3/CH_3OH$: 3:1, Kieselgel auf Alufolie/Merck 5562
$R_f$ = 0,71
Ausbeute: 12,1 g (44,6 % d.Th.)

Beispiel 4

1-Acetyl-5-fluor-2-methyl-3-indolsuccinanhydrid

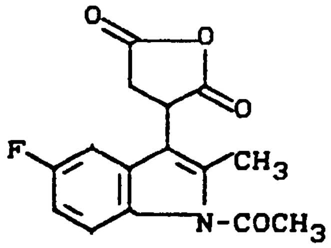

Die Titelverbindung wurde analog der Vorschrift von Beispiel 3 hergestellt.
Fp.: 178°C
DC : $CHCl_3/CH_3OH$ = 3 : 1, Kieselgel auf Alufolie/Merck 5562
$R_f$ = 0,65
Ausbeute: 47,6 %

Beispiel 5

1-Acetyl-2-methyl-1,3,4,5-tetrahydro-5-oxo-benz-[c,d]-indol-3-carbonsäure

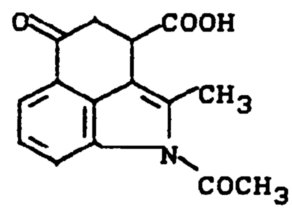

2,71 g (0,01 mol) der Verbindung aus Beispiel 3 werden in 60 ml warmem Ethylendichlorid gelöst. Die Lösung wird unter Rühren gekühlt, bis sich eine feine Suspension zeigt. Zu einer Suspension von 60 ml Anhydrid in 60 ml Ethylendichlorid werden bei Raumtemperatur 6,67 g (0,05 mol) Aluminiumchlorid zugefügt. Die Lösung wird 4 h auf 40°C erhitzt, gekühlt und mit 50 ml 2 N HCl versetzt. Der Rückstand wird abfiltriert, mit Wasser gewaschen und in warmem Aceton gelöst. Die Lösung wird mit Aktivkohle behandelt und durch Kühlung kristallisiert.

Fp.: 215-217°C
DC: $CHCl_3/CH_3OH$ = 3:1, Kieselgel auf Alufolie/Merck 5562
$R_f$ = 0,50
Ausbeute: 0,75 g (27,7 %)

Beispiel 6

1-Acetyl-6-fluor-2-methyl-1,3,4,5-tetrahydro-5-oxo-benz-[c,d]-indol-3-carbonsäure

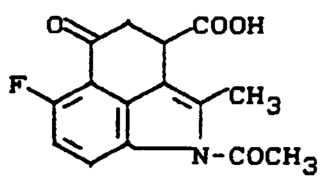

Die Titelverbindung wurde analog der Vorschrift von Beispiel 5 aus 0,27 (0,01 mol) der Verbindung aus Beispiel 4 hergestellt.

Fp.: 248°C (Zersetzung)
DC : $CHCl_3/CH_3OH$ = 3 : 1, Kieselgel auf Alufolie/Merck 5562
$R_f$ = 0,36
Ausbeute: 82,4 %

Beispiel 7

2-Methyl-1,3,4,5-tetrahydro-5-oxobenz-[c,d]-indol-3-carbonsäure

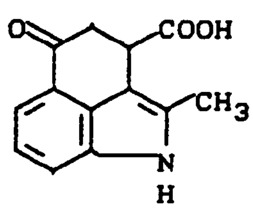

Eine Lösung aus 1 g (0,0037 mol) der Verbindung aus Beispiel 5 und 50 ml 0,2 N NaOH wird 3 h bei Raumtemperatur stehen gelassen und danach mit verdünnter Salzsäure angesäuert. Das ausgefallene Produkt wird abfiltriert, mit Wasser gewaschen und aus verdünnter Essigsäure umkristallisiert.

Fp.: 235-239°C
DC: $CHCl_3/CH_3OH$ = 3:1, Kieselgel auf Alufolie/Merck 5562
$R_f$: 0,50
Ausbeute: 0,60 g (71,4 % d.Th.)

Beispiel 8

2-Methyl-1,3,4,5-tetrahydrobenz-[c,d]-indol-3-carbonsäure

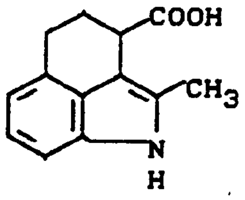

22,9 g (0,1 mol) der Verbindung aus Beispiel 7 und 0,5 Mol Hydrazinhydrat (85 %-Lösung) werden bei Raumtemperatur zu einer gekühlten Lösung von NaOH (0,5 mol) in 250 ml Diethylenglykol gegeben. Die Mischung wird 20 Min. unter Rückfluß erhitzt, gekühlt, mit 500 ml Wasser verdünnt und mit Ether extrahiert.

Die wäßrige Lösung wird in einem Eisbad gekühlt, mit HCl konz. angesäuert und mit Ether extrahiert. Die etherische Lösung wird mit Wasser gewaschen, über Magnesiumsulfat getrocknet und im Vakuum eingeengt.

Der Rückstand wird in Chloroform gelöst und mit Aktivkohle behandelt; diese wird anschließend abfiltriert und die Lösung eingeengt und gekühlt. Das Produkt fällt in gelben Plättchen aus.

Fp.: 103°C
DC: Toluol/$C_2H_5OH$ = 3:1, Kieselgel auf Alufolie/Merck 5562
$R_f$: 0,53
Ausbeute: 14,2 g (66,0 % d.Th.)

Beispiel 9

6-Fluor-2-methyl-1,2,4,5-tetrahydrobenz-[c,d]-indol-3-carbonsäure

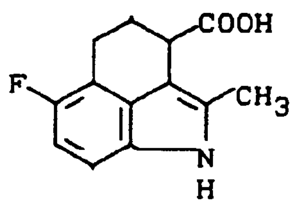

Die Titelverbindung wird analog der Vorschrift von Beispiel 8 mit 22,9 g (0,1 mol) der Verbindung aus Beispiel 6 hergestellt.

Fp.: 190°C
DC : Toluol/Ethanol = 3 : 1, Kieselgel auf Alufolie/Merck 5562
$R_f$ : 0,55
Ausbeute: 53,8 %

Beispiel 10

2-Methyl-1,3,4,5-tetrahydrobenz-[c,d]-indol-3-carbonsäureamid

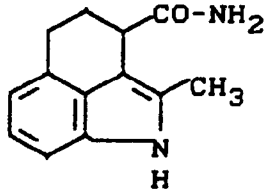

4,30 g (0,02 mol) der Verbindung aus Beispiel 8 werden in 50 ml Tetrahydrofuran abs. gelöst und mit 3,89 g (0,024 mol) N,N'-Carbonyldiimidazol versetzt. Es wird über Nacht gerührt, eingeengt und der Rückstand in Methylenchlorid aufgenommen. Danach wird 2 mal mit Wasser gewaschen, über Magnesiumsulfat getrocknet, eingeengt und der Rückstand in 50 ml Tetrahydrofuran abs. gelöst. Anschließend wird mit 20 ml 5 N methanolischer Ammoniak-Lösung versetzt. Es wird 2 h bei Raumtemperatur gerührt, eingeengt und das Produkt aus Essigester kristallisiert.

Fp.: 218°C
DC = $CH_2Cl_2$/Essigester 1:1, Kieselgel auf Alufolie/Merck 5562
$R_f$ = 0,21
Ausbeute: 3,50 g (81,8 % d.Th.)

Beispiel 11

6-Fluor-2-methyl-1,3,4,5-tetrahydrobenz-[c,d]-indol-3-carbonsäureamid

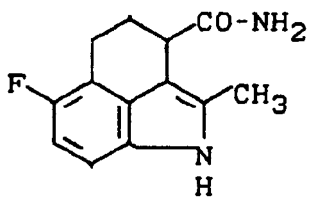

Die Titelverbindung wird analog der Vorschrift von Beispiel 10 aus 4,30 g (0,02 mol) der Verbindung aus Beispiel 9 hergestellt.

Fp.: 230°C
DC : $CH_2Cl_2$/Essigester 1 : 1, Kieselgel auf Alufolie/Merck 5562
$R_f$ : 0,20
Ausbeute: 68,8 %

Beispiel 12

3-Aminomethyl-2-methyl-1,3,4,5-tetrahydrobenz-[c,d]-indol

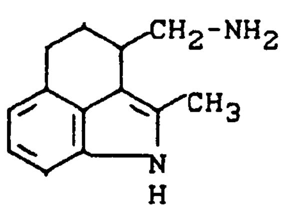

3,21 g (0,015 mol) der Verbindung aus Beispiel 10 werden in 70 ml Tetrahydrofuran abs. suspendiert. Dann tropft man bei Raumtemperatur 30 ml einer 1-molaren (0,03 mol) Lithiumaluminiumhydrid-Lösung in Tetrahydrofuran, zu. Danach wird 2 h bei Rückflußtemperatur gerührt, unter Eiskühlung tropfenweise mit 20 ml Essigester, 2 ml Wasser und 4 ml KOH (15 %ig) versetzt und 30 Min. gerührt. Die Salze werden abfiltriert. Nach dem Einengen wird der Rückstand in Methylenchlorid aufgenommen, mit Wasser gewaschen, getrocknet und wieder eingedampft. Der Rückstand wird mit Hilfe der Flash-Chromatographie an Kieselgel 60-Merck ($CH_2Cl_2$/Essigester 1:1, $CH_2Cl_2$/$CH_3OH$ 1:1) gereinigt.

gelbbraunes Harz
DC = $CH_2Cl_2$/$CH_3OH$ 1:1, Kieselgel auf Alufolie/Merck 5562
$R_f$ = 0,16
Ausbeute: 2,80 g (93 % d.Th.)

Beispiel 13

3-Aminomethyl-6-fluor-2-methyl-1,3,4,5-tetrahydrobenz-[c,d]-indol

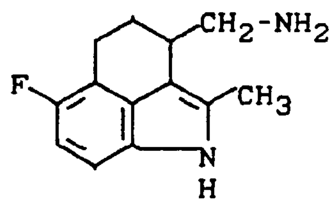

Die Titelverbindung wurde analog der Vorschrift von Beispiel 12 aus 3,21 g (0,015 mol) der Verbindung aus Beispiel 11 hergestellt.

Fp.: 230°C
DC: $CH_2Cl_2$/Essiigester 1 : 1, Kieselgel auf Alufolie/Merck 5562
$R_f$ = 0,20
Ausbeute: 68,8 %

Beispiel 14

2-Methyl-3-(4-toluolsulfonyl)-aminomethyl-1,3,4,5-tetrahydro-benz-[c,d]-indol

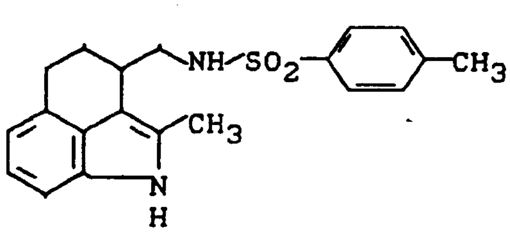

3,00 g (0,015 mol) 3-Aminomethyl-2-methyl-1,3,4,5-tetrahydro-benz-[c,d]-indol und 5 ml Triethylamin werden in 100 ml Methylenchlorid gelöst. Danach werden 2,86 g (0,015 mol) p-Toluolsulfonsäurechlorid gelöst und in 40 ml Methylenchlorid innerhalb 30 Minuten zugetropft. Es wird 2 Stunden bei Raumtemperatur nachgerührt. Das Reaktionsgemisch wird mit 1 N Salzsäure, dann mit Wasser ausgeschüttelt. Die organische Phase wird über Magnesiumsulfat getrocknet und im Vakuum eingedampft. Den erhaltenen Rückstand bringt man mit Isopropanol zur Kristallisation. Der Niederschlag wird abgesaugt, mit Isopropanol und Diethylether gewaschen und im Hochvakuum bei 50°C getrocknet.

Ausbeute: 3,96 g (74,7 % der Theorie)
Schmelzpunkt: 211°C
$R_f$-Wert: 0,48 (Kieselgel auf Aluminiumfolie Merck 5562, Laufmittel Toluol/Essigester 3/1)

Die in den folgenden Tabellen aufgeführten Beispiele wurden in Analogie zu Beispiel 14 hergestellt.

Tabelle 1

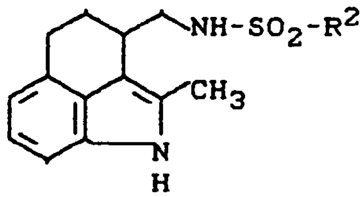

| Bsp. | $R^2$ | Fp: | $R_f$ (Toluol/Essigester = 3 : 1) | |
|---|---|---|---|---|
| 15 | 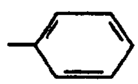 | 152° C | 0,51 | Kieselgel auf Alufolie Merck 5562 |
| 16 | 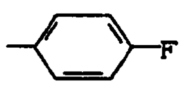 | 156° C | 0,49 | |
| 17 | 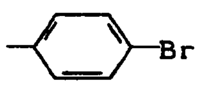 | 214° C | 0,57 | |
| 18 | 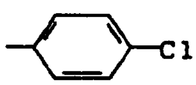 | 201° C | 0,52 | |
| 19 | 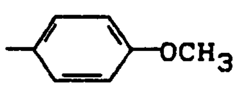 | 191° C | 0,41 | |
| 20 | 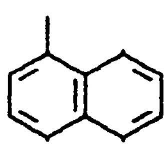 | 128° C | 0,57 | |

Tabelle 2

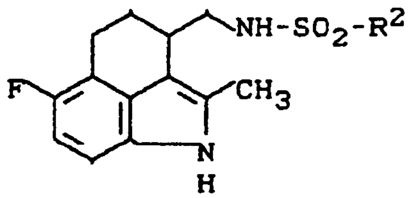

| Bsp. | $R^2$ | Fp: | $R_f$ (Toluol/Essigester = 3 : 1) | |
|---|---|---|---|---|
| 21 | 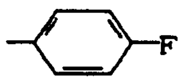 | 123° C | 0,40 | Kieselgel auf Alufolie Merck 5562 |
| 22 | 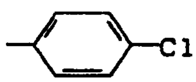 | 156° C | 0,50 | |
| 23 | 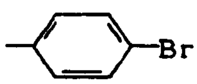 | 161° C | 0,47 | |
| 24 | 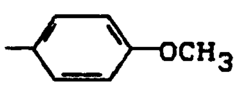 | 148° C | 0,35 | |
| 25 | 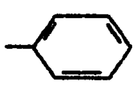 | 170° C | 0,41 | |
| 26 | 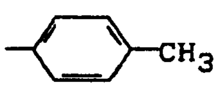 | 173° C | 0,44 | |

Beispiel 27

1-(2-Cyanethyl)-3-[N-(2-cyanethyl)-N-(4-toluolsulfonyl)-aminomethyl]-2-methyl-1,3,4,5-tetrahydro-benz-[c,d]-indol

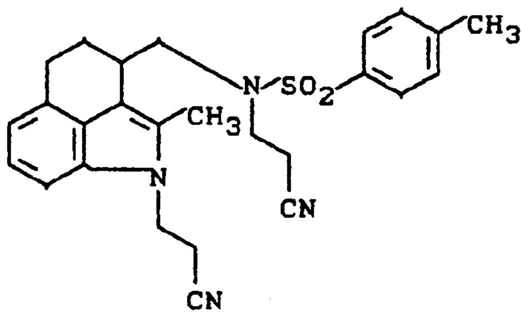

3,86 g (0,011 mol) 2-Methyl-3-(4-toluolsulfonyl)-aminomethyl-1,3,4,5-tetrahydro-benz-[c,d]-indol werden in 50 ml absolutem 1,4-Dioxan gelöst und nacheinander, mit 4 ml Acrylnitril und 4 Tropfen Benzyltrimethylammoniumhydroxidlösung (40 %ig in Methanol) versetzt. Es wird 3 Stunden bei 80°C nachgerührt. Der Ansatz wird nach Abdampfen des Lösungsmittels in Essigester aufgenommen und 2mal mit Wasser ausgeschüttelt. Die organische Phase wird über Magnesiumsulfat getrocknet und eingedampft. Der Rückstand wird mit Methanol zur Kristallisation gebracht. Anschließend wird der Niederschlag abgesaugt, mit Methanol gewaschen und im Hochvakuum bei 50°C getrocknet.

Ausbeute: 4,47 g (89,2 % der Theorie)

Schmelzpunkt: 163°C

$R_f$-Wert: 0,20 (Kieselgel auf Aluminiumfolie Merck 5562, Laufmittel Toluol/Essigester 6/1)

Die in den folgenden Tabellen aufgeführten Beispiele werden in Analogie zu Beispiel 27 hergestellt:

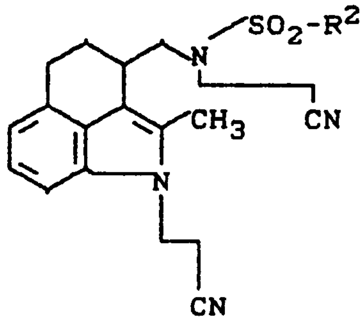

| Bsp. | $R^2$ | Fp: | $R_f$ (Toluol/Essigester = 6 : 1) | |
|---|---|---|---|---|
| 28 | 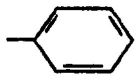 | 178° C | 0,18 | Kieselgel auf Alufolie Merck 5562 |
| 29 | 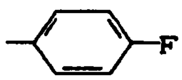 | 206° C | 0,17 | |
| 30 | 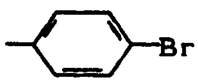 | 140° C | 0,24 | |
| 31 | 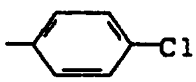 | 124° C | 0,21 | |
| 32 | 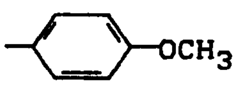 | 146° C | 0,13 | |
| 33 | 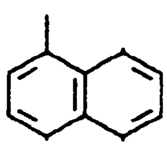 | 102° C | 0,22 | |

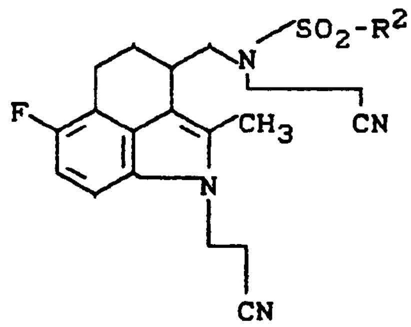

| Bsp. | $R^2$ | Fp: | $R_f$ (Toluol/Essigester = 6 : 1) | |
|---|---|---|---|---|
| 34 | 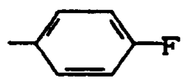 | 153° C | 0,28 | Kieselgel auf Alufolie Merck 5562 |
| 35 | 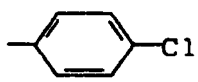 | 130° C | 0,32 | |
| 36 | 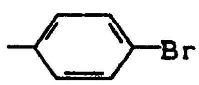 | 128° C | 0,33 | |
| 37 | 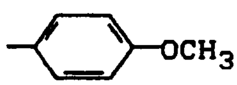 | 152° C | 0,19 | |
| 38 | 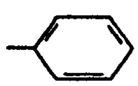 | 166° C | 0,20 | |
| 39 | 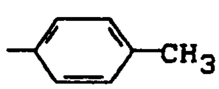 | 118° C | 0,29 | |

Beispiel 40

2-Methyl-3-(4-toluolsulfonyl)-aminomethyl-1,3,4,5-tetrahydro-1-benz-[c,d]-indolpropionsäure

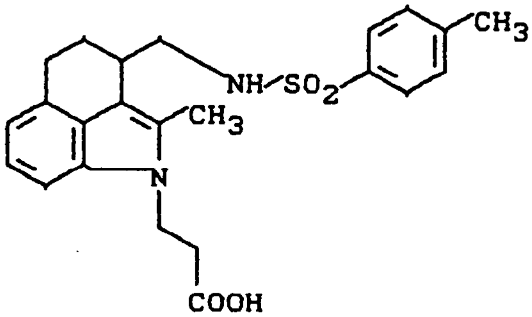

4,38 g (0,0095 mol) 1-(2-Cyanethyl)-3-[N-(2-cyanethyl)-N-(4-toluolsulfonyl)-aminomethyl]-2-methyl-1,3,4,5-tetrahydro-benz-[c,d]-indol werden mit 15 g Kaliumhydroxid und 85 ml Wasser versetzt und 6 Stunden unter Rückfluß kräftig gerührt. Der Ansatz wird anschließend unter Eiskühlung mit 6 N Salzsäure sauer gestellt und danach 3mal mit Methylenchlorid extrahiert. Die vereinigten organischen Phasen werden 1 mal mit Wasser gewaschen, über Magnesiumsulfat getrocknet und im Vakuum eingedampft. Der Rückstand wird mit Toluol unter Rühren zur Kristallisation gebracht. Es wird abgesaugt, mit Toluol und n-Pentan gewaschen und im Hochvakuum bei 50°C getrocknet.

Ausbeute: 4,05 g (100 % der Theorie)

Schmelzpunkt: 154°C

$R_f$-Wert: 0,54 (Kieselgel auf Aluminiumfolie Merck 5562, Laufmittel Toluol/Ethanol 3/1)

Die in den folgenden Tabellen aufgeführten Beispiele wurden in Analogie zu Beispiel 40 hergestellt:

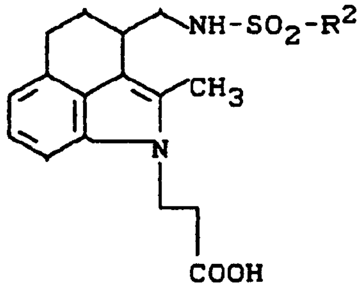

| Bsp. | $R^2$ | Fp: | $R_f$ (Toluol/Essigester = 3 : 1) | |
|---|---|---|---|---|
| 41 | 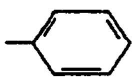 | 118° C | 0,54 | Kieselgel auf Alufolie Merck 5562 |
| 42 | 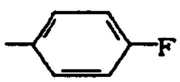 | 154° C | 0,58 | |
| 43 | 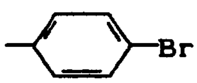 | 162° C | 0,56 | |
| 44 | 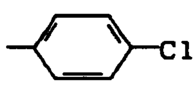 | 172° C | 0,50 | |
| 45 | 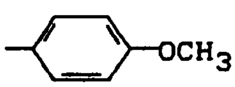 | 104° C | 0,51 | |
| 46 | 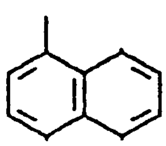 Na-Salz | 66° C | 0,53 | |

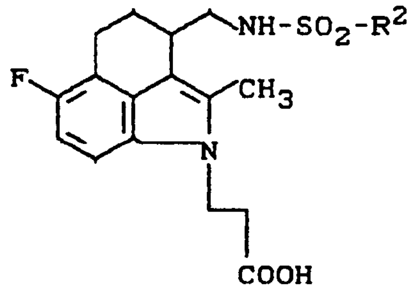

| Bsp. | $R^2$ | Fp: | $R_f$ (Toluol/Essigester = 3 : 1) | |
|---|---|---|---|---|
| 47 | 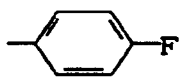 | 124° C | 0,47 | Kieselgel auf Alufolie Merck 5562 |
| 48 | 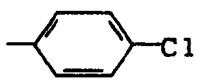 | 115° C | 0,53 | |
| 49 | 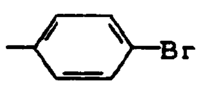 | 108° C | 0,45 | |
| 50 | 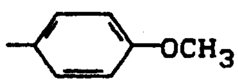 | 155° C | 0,46 | |
| 51 | 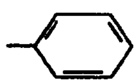 | 157° C | 0,44 | |
| 52 | 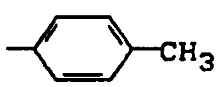 | 157° C | 0,49 | |

Anwendungsbeispiel 53

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37°C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet. Als Grenzkonzentration wird der Bereich der minimal effektiven Konzentration angegeben.

Die Grenzkonzentrationen liegen zwischen 0,003 - 10 mg/l

Zum Beispiel 18 als Beispiel:

EC = 0,01 - 0,003 mg/l.

**Patentansprüche**

**Patentansprüche für folgende Verstragsstaaten : AT, BE, CH, DE, FR, GB, GR, IT, LI, LU, NL, SE**

1. Tetrahydro-1-benz-[c,d]-indol-propionsäure-sulfonamide der Formel

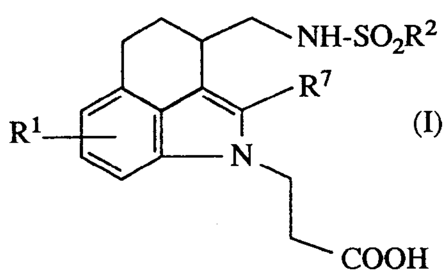

(I)

worin

$R^1$

– für Wasserstoff, fluor, Chor, Brom, Trifluormethyl, Carboxy oder $C_1$-$C_6$-Alkoxycarbonyl steht,

oder

– für eine Gruppe -$S(O)_nR^3$, $NR^4R^5$ oder -$OR^6$ steht,

worin

$R^3$ - $C_1$-$C_6$-Alkyl oder Phenyl bedeutet,

$R^4$ und $R^5$ gleich oder verschieden sind und Wasserstoff, $C_1$-$C_6$-Akyl, Phenyl oder Benzyl bedeuten,

$R^6$ - Wasserstoff, $C_1$-$C_6$-Alkyl, Phenyl, Benzyl oder Trifluormethyl bedeutet,

und

n - eine Zahl 0 oder 2 bedeutet,

oder

$R^1$ - für $C_1$-$C_6$-Alkyl, $C_2$-$C_6$-Alkenyl, Cyclopropyl, Cyclopentyl oder Cyclohexyl steht, das gegebenenfalls durch Carboxy, $C_1$-$C_6$-Alkoxy, Fluor, Chor, Brom, Hydroxy, $C_1$-$C_6$-Alkoxycarbonyl, $C_1$-$C_6$-Alkylthio oder Cyano substituiert ist,

$R^2$ - für Phenyl oder Naphthyl steht, das bis zu 3-fach gleich oder verschieden durch Fluor, Chor, Brom, Cyano, Trifluormethyl, Trifluormethoxy, Trifluormethylthio, $C_1$-$C_6$-Alkyl, Carboxymethyl, Carboxyethyl, Methoxycarbonylmethyl, Ethoxycarbonylmethyl, Methoxycarbonylethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio

oder durch eine Gruppe der Formel -$NR^4R^5$ substituiert ist,

worin

$R^4$ und $R^5$ die oben angegebene Bedeutung haben,

$R^7$ - für Wasserstoff, $C_1$-$C_6$-Alkyl, Cyclopropyl, Cyclopentyl oder Cyclohexyl steht,

und deren Salze.

2. Tetrahydro-1-benz-[c,d]-indolpropionsäure-sulfonamide nach Anspruch 1

worin

$R^1$ - für Wasserstoff, fluor, Chlor, Brom, Trifluormethyl, Methylthio, Ethylthio, Methylsulfonyl, Ethylsulfonyl, Phenylthio, Phenylsulfonyl, Amino, Dimethylamino, Diethylamino steht,

oder

– für eine Gruppe -$OR^6$ steht,

worin

$R^6$ - Wasserstoff, Methyl, Ethyl, Propyl, Phenyl oder Benzyl bedeutet

oder

– für Methyl, Ethyl, Propyl oder Isopropyl steht,

$R^2$ - für Phenyl oder Naphthyl steht, das bis zu 2-fach gleich oder verschieden durch Fluor, Chor, Brom, Cyano, Trifluormethyl, Trifluormethoxy, Methyl, Ethyl, Propyl, Methoxy, Ethoxy, Propoxy, Methylthio, Hydroxy, Methoxycarbonyl, Ethoxycarbonyl, Phenyl, Dimethylamino oder Diethylamino substituiert ist,

$R^7$ - für Wasserstoff oder Methyl steht, und deren Salze.

**3.** Tetrahydro-1-benz-[c,d]-indolpropionsäure-sulfonamide nach den Ansprüchen 1 und 2 worin

$R^1$ - Wasserstoff oder Fluor bedeutet,

$R^2$ - für Phenyl oder Naphthyl steht, das durch Fluor, Chlor, Brom, Methyl, Methoxy oder Phenyl substituiert ist,

$R^7$ - für Methyl steht,

und deren Salze.

**4.** Tetrahydro-1-benz-[c,d]-indolpropionsäure-sulfonamide nach den Ansprüchen 1 bis 3 zur therapeutischen Behandlung.

**5.** Verfahren zur Herstellung von Tetrahydro-1-benz-[c,d]-indolpropionsäure-sulfonamide nach Anspruch 1, dadurch gekennzeichnet, daß man Tetrahydro-1-benz-[c,d]-indolsulfonamide der Formel

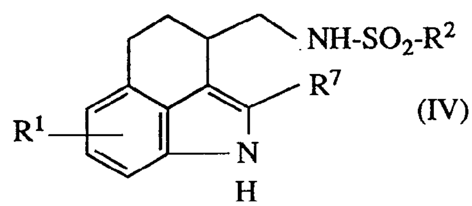

(IV)

in welcher

$R^1$, $R^2$ und $R^7$ die in Anspruch 1 angegebene Bedeutung haben,

mit Acrylnitril der Formel (V)

$$H_2C = CH - CN \qquad (V)$$

in Gegenwart eines inerten Lösemittels, gegebenenfalls in Gegenwart einer Base zu den Verbindungen der allgemeinen Formel (VI)

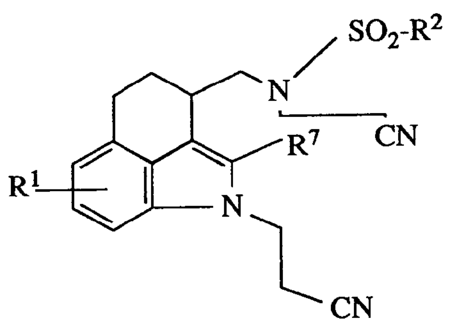

(VI)

in welcher

$R^1$, $R^2$ und $R^7$ die in Anspruch 1 angegebene Bedeutung haben

umsetzt

und die N,N'-Biscyanoethylverbindungen (VI) anschließend verseift.

**6.** Verfahren nach Anspruch 5, dadurch gekennzeichnet, daß es im Temperaturbereich von 0 bis 150°C durchgeführt wird.

7. Tetrahydro- 1-benz-[c,d]-indol-sulfonamide der Formel

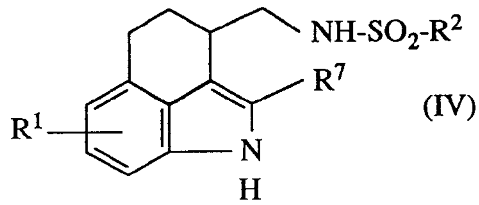

(IV)

in welcher $R^1$

– für Wasserstoff, Fluor, Chlor, Brom, Trifluormethyl, Carboxy oder $C_1$-$C_6$-Alkoxycarbonyl steht, oder

– für eine Gruppe -$S(O)_nR^3$, $NR^4R^5$ oder -$OR^6$ steht,

worin

$R^3$ - $C_1$-$C_6$-Akyl oder Phenyl bedeutet,

$R^4$ und $R^5$ gleich oder verschieden sind und Wasserstoff, $C_1$-$C_6$-Alkyl, Phenyl oder Benzyl bedeuten,

$R^6$ - Wasserstoff, $C_1$-$C_6$-Akyl, Phenyl, Benzyl oder Trifluormethyl bedeutet,

und

n - eine Zahl 0 oder 2 bedeutet,

oder

$R^1$ - für $C_1$-$C_6$-Alkyl, $C_2$-$C_6$-Alkenyl, Cyclopropyl, Cyclopentyl oder Cyclohexyl steht, das gegebenenfalls durch Carboxy, $C_1$-$C_6$-Alkoxy, Fluor, Chor, Brom, Hydroxy, $C_1$-$C_6$-Alkoxycarbonyl, $C_1$-$C_6$-Alkylthio oder Cyano substituiert ist,

$R^2$ - für Phenyl oder Naphthyl steht, das bis zu 3-fach gleich oder verschieden durch Fluor, Chlor, Brom, Cyano, Trifluormethyl, Trifluormethoxy, Trifluomethylthio, $C_1$-$C_6$-Alkyl, Carboxymethyl, Carboxyethyl, Methoxycarbonylmethyl, Ethoxycarbonylmethyl, Methoxycarbonylethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio

oder durch eine Gruppe der Formel -$NR^4R^5$ substituiert ist,

worin

$R^4$ und $R^5$ die oben angegebene Bedeutung haben,

$R^7$ - für Wasserstoff, $C_1$-$C_6$-Alkyl, Cyclopropyl, Cyclopentyl oder Cyclohexyl steht,

und deren Salze.

8. Verfahren zur Herstellung von Tetrahydro-1-benz-[c,d]-indolsulfonamide nach Anspruch 7, dadurch gekennzeichnet, daß man Indole der Formel

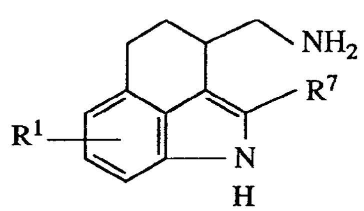

(VII)

in welcher

$R^1$ und $R^7$ die in Anspruch 7 angegebene Bedeutung haben mit Sulfonsäurehalogeniden der allgemeinen Formel (VIII)

$$X - SO_2 - R^2 \qquad (VIII)$$

in welcher

$R^2$ die in Anspruch 7 angegebene Bedeutung hat,

und

X- für Halogen steht,

in inerten Lösemitteln, gegebenenfalls in Anwesenheit einer Base umsetzt.

9. Arzneimittel enthaltend Tetrahydro-1-benz-[c,d]-indol-propionsäure-sulfonamide nach den Ansprüchen 1 bis 3.

10. Arzneimittel nach Anspruch 9, enthaltend 0,5 bis 90 Gew.-% Tetrahydro-1-benz-[c,d]-indol-propionsäure-sulfonamide, bezogen auf die Zubereitung.

11. Verwendung von Tetrahydro-1-benz- [c,d] -indol-propionsäure-sulfonamide nach den Ansprüchen 1 bis 3 zur Herstellung von Arzneimitteln.

**Patentansprüche für folgenden Verstragsstaat : ES**

1. Verfahren zur Herstellung von Tetrahydro-1-benz-[c,d]-indol-propionsäure-sulfonamide der Formel

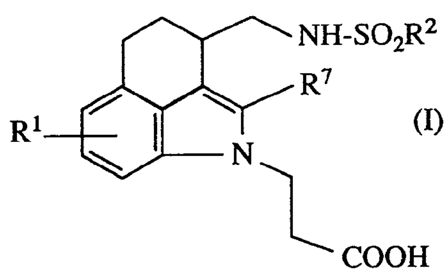

(I)

worin

$R^1$

– für Wasserstoff, Fluor, Chor, Brom, Trifluormethyl, Carboxy oder $C_1$-$C_6$-Alkoxycarbonyl steht, oder

– für eine Gruppe -$S(O)_nR^3$, $NR^4R^5$ oder -$OR^6$ steht,

worin

$R^3$ - $C_1$-$C_6$-Akyl oder Phenyl bedeutet,

$R^4$ und $R^5$ gleich oder verschieden sind und Wasserstoff, $C_1$-$C_6$-Alkyl, Phenyl oder Benzyl bedeuten,

$R^6$ - Wasserstoff, $C_1$-$C_6$-Akyl, Phenyl, Benzyl oder Trifluormethyl bedeutet,

und

n - eine Zahl 0 oder 2 bedeutet,

oder

$R^1$ - für $C_1$-$C_6$-Alkyl, $C_2$-$C_6$-Alkenyl, Cyclopropyl, Cyclopentyl oder Cyclohexyl steht, das gegebenenfalls durch Carboxy, $C_1$-$C_6$-Alkoxy, Fluor, Chor, Brom, Hydroxy, $C_1$-$C_6$-Alkoxycarbonyl, $C_1$-$C_6$-Alkylthio oder Cyano substituiert ist,

$R^2$ - für Phenyl oder Naphthyl steht, das bis zu 3-fach gleich oder verschieden durch Fluor, Chor, Brom, Cyano, Trifluormethyl, Trifluormethoxy, Trifluormethylthio, $C_1$-$C_6$-Akyl, Carboxymethyl, Carboxyethyl, Methoxycarbonylmethyl, Ethoxycarbonylmethyl, Methoxycarbonylethyl, $C_1$-$C_6$-Akoxy, $C_1$-$C_6$-Alkylthio, Hydroxy, Carboxy, $C_1$-$C_6$-Alkoxycarbonyl, Phenyl, Phenoxy, Benzyloxy, Benzylthio

oder durch eine Gruppe der Formel -$NR^4R^5$ substituiert ist,

worin

$R^4$ und $R^5$ die oben angegebene Bedeutung haben,

$R^7$ - für Wasserstoff, $C_1$-$C_6$-Alkyl, Cyclopropyl, Cyclopentyl oder Cyclohexyl steht,

und deren Salze,

dadurch gekennzeichnet, daß man Tetrahydro-1-benz-[c,d]-indol-sulfonamide der Formel

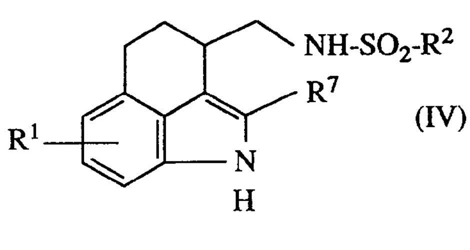

(IV)

in welcher

$R^1$, $R^2$ und $R^7$ die oben angegebene Bedeutung haben,

mit Acrylnitril der Formel (V)

$$H_2C = CH - CN \qquad (V)$$

in Gegenwart eines inerten Lösemittels, gegebenenfalls in Gegenwart einer Base zu den Verbindungen der allgemeinen Formel (VI)

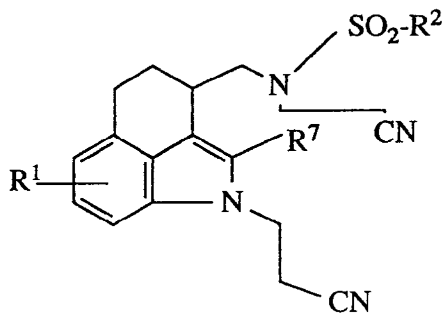

(VI)

in welcher

$R^1$, $R^2$ und $R^7$ die oben angegebene Bedeutung haben

umsetzt

und die N,N'-Biscyanoethylverbindungen (VI) anschießend verseift.

2. Verfahren nach Anspruch 1, dadurch gekennzeichnet, daß es im Temperaturbereich von 0 bis 150°C durchgeführt wird.

3. Verfahren nach Anspruch 1, dadurch gekennzeichnet, daß man die Verseifung der N,N'-Bis-cyanoethylverbindungen in einem Temperaturbereich von 0°C bis +100°C durchführt.

**Claims**

**Claims for the following Contracting States : AT, BE, CH, DE, FR, GB, GR, IT, LI, LU, NL, SE**

1. Tetrahydro-1-benz-[c,d]-indole-propionic acid sulphonamides of the formula

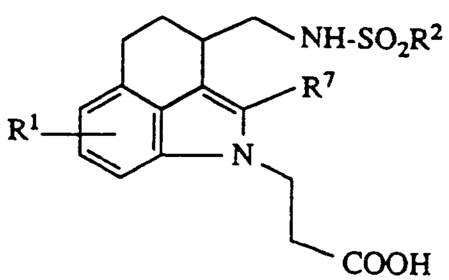

(I)

in which

$R^1$

– represents hydrogen, fluorine, chlorine, bromine, trifluoromethyl, carboxyl or $C_1$-$C_6$-alkoxycarbonyl,

or

– represents a group -$S(O)_nR^3$, $NR^4R^5$ or -$OR^6$,

in which

$R^3$ - denotes $C_1$-$C_6$-alkyl or phenyl,

$R^4$ and $R^5$ are identical or different and denote hydrogen, $C_1$-$C_6$-alkyl, phenyl or benzyl,

$R^6$ - denotes hydrogen, $C_1$-$C_6$-alkyl, phenyl, benzyl or trifluoromethyl,

n - denotes a number 0 or 2,

or

$R^1$ - represents $C_1$-$C_6$-alkyl, $C_2$-$C_6$-alkenyl, cyclopropyl, cyclopentyl or cyclohexyl, which is optionally substituted by carboxyl, $C_1$-$C_6$-alkoxy, fluorine, chlorine, bromine, hydroxyl, $C_1$-$C_6$-alkoxycarbonyl, $C_1$-$C_6$-alkylthio or cyano,

$R^2$ - represents phenyl or naphthyl, which is mono-substituted to trisubstituted by identical or different substituents from the series consisting of fluorine, chlorine, bromine, cyano, trifluoromethyl, trifluoromethoxy, trifluoromethylthio, $C_1$-$C_6$-alkyl, carboxyethyl, carboxyethyl, methoxycarbonylmethyl, ethoxycarbonylmethyl, methoxycarbonylethyl,$C_1$-$C_6$-alkoxy, $C_1$-$C_6$-alkylthio, hydroxyl, carboxyl, $C_1$-$C_6$-alkoxycarbonyl,phenyl,phenoxy,benzyloxy or benzylthio,

or by a group of the formula $-NR^4R^5$,

in which

$R^4$ and $R^5$ have the abovementioned meaning,

$R^7$ - represents hydrogen, $C_1$-$C_6$-alkyl, cyclopropyl, cyclopentyl or cyclohexyl,

and their salts.

**2.** Tetrahydro-1-benz-[c,d]-indolepropionic acid sulphonamides according to Claim 1,

in which

$R^1$

– represents hydrogen, fluorine, chlorine, bromine, trifluoromethyl, methylthio, ethylthio, methylsulphonyl, ethylsulphonyl, phenylthio, phenylsulphonyl, amino, dimethylamino or diethylamino,

or

– represents a group $OR^6$,

in which

$R^6$ - denotes hydrogen, methyl, ethyl, propyl, phenyl or benzyl

or

– represents methyl, ethyl, propyl or isopropyl,

$R^2$ - represents phenyl or naphthyl, which is monosubstituted or disubstituted by identical or different substituents from the series consisting of fluorine, chlorine, bromine, cyano, trifluoromethyl, trifluoromethoxy, methyl, ethyl, propyl, methoxy, ethoxy, propoxy, methylthio, hydroxyl, methoxycarbonyl, ethoxycarbonyl, phenyl, dimethylamino or diethylamino,

$R^7$ - represents hydrogen or methyl, and their salts.

**3.** Tetrahydro-1-benz-[c,d]-indolepropionic acid sulphonamides according to Claims 1 and 2

in which

$R^1$ - denotes hydrogen or fluorine,

$R^2$ - represents phenyl or naphthyl, which is substituted by fluorine, chlorine, bromine, methyl, methoxy or phenyl,

$R^7$ - represents methyl,

and their salts

**4.** Tetrahydro-1-benz-[c,d]-indolepropionic acid sulphonamides according to Claims 1 to 3 for therapeutic treatment.

**5.** Process for the preparation of tetrahydro-1-benz-[c,d]-indolepropionic acid sulphonamides according to Claim 1,

characterised in that tetrahydro-1-benz-[c,d]-indolesulphonamides of the formula

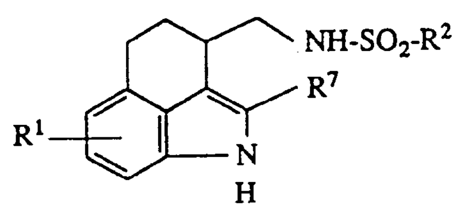

(IV)

in which

$R^1$, $R^2$ and $R^7$ have the meaning given in Claim 1, are reacted with acrylonitrile of the formula (V)

$$H_2C=CH-CN \qquad (V)$$

in the presence of an inert solvent and if appropriate in the presence of a base to give the compounds of the general formula (VI)

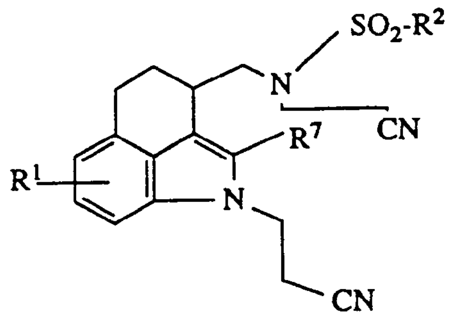

(VI)

in which

$R^1$, $R^2$ and $R^7$ have the meaning given in Claim 1 and the N,N'-biscyanoethyl compounds (VI) are then hydrolysed.

6. Process according to Claim 5, characterised in that it is carried out in the temperature range from 0 to 150°C.

7. Tetrahydro-1-benz-[c,d]-indole-sulphonamides of the formula

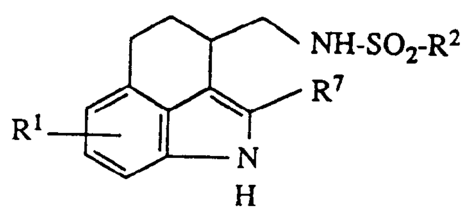

(IV)

in which

$R^1$

- represents hydrogen, fluorine, chlorine, bromine, trifluoromethyl, carboxyl or $C_1$-$C_6$-alkoxycarbonyl, or
- represents a group $-S(O)_nR^3$, $NR^4R^5$ or $-OR^6$,

in which

$R^3$ - denotes $C_1$-$C_6$-alkyl or phenyl,

$R^4$ and $R^5$ are identical or different and denote hydrogen, $C_1$-$C_6$-alkyl, phenyl or benzyl,

$R^6$ - denotes hydrogen, $C_1$-$C_6$-alkyl, phenyl, benzyl or trifluoromethyl,

and

n - denotes a number 0 or 2,

or

$R^1$ - represents $C_1$-$C_6$-alkyl, $C_2$-$C_6$-alkenyl, cyclopropyl, cyclopentyl or cyclohexyl, which is optionally substituted by carboxyl, $C_1$-$C_6$-alkoxy, fluorine, chlorine, bromine, hydroxyl, $C_1$-$C_6$-alkoxycarbonyl, $C_1$-$C_6$-alkylthio or cyano,

$R^2$ - represents phenyl or naphthyl, which is mono-substituted to trisubstituted by identical or different substituents from the series consisting of fluorine, chlorine, bromine, cyano, trifluoromethyl, trifluoromethoxy, trifluoromethylthio, $C_1$-$C_6$-alkyl, carboxyethyl, carboxyethyl, methoxycarbonylmethyl, ethoxycarbonylmethyl, methoxycarbonylethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy or benzylthio,

or by a group of the formula $-NR^4R^5$,

in which

$R^4$ and $R^5$ have the abovementioned meaning,

$R^7$ - represents hydrogen, $C_1$-$C_6$-alkyl, cyclopropyl, cyclopentyl or cyclohexyl,

and their salts

8. Process for the preparation of tetrahydro-1-benz-[c,d]-indolesulphonamides according to Claim 7 characterised in that indoles of the formula

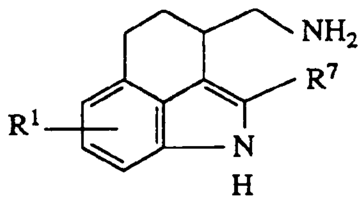

(VII)

in which

$R^1$ and $R^7$ have the meaning given in Claim 7

are reacted with sulphonyl halides of the general formula (VIII)

$X-SO_2-R^2$ (VIII)

in which

$R^2$ has the meaning given in Claim 7,

and

X - represents halogen,

in inert solvents, if appropriate in the presence of a base.

9. Medicaments containing tetrahydro-1-benz-[c,d]-indole-propionic acid sulphonamides according to Claims 1 to 3.

10. Medicaments according to Claim 9, containing 0.5 to 90% by weight of tetrahydro-1-benz-[c,d]-indole-propionic acid sulphonamides, relative to the preparation.

11. Use of tetrahydro-1-benz-[c,d]-indole-propionic acid sulphonamides according to Claims 1 to 3 for the production of medicaments.

**Claims for the following Contracting State : ES**

1. Process for the preparation of tetrahydro-1-benz-[c,d]-indole-propionic acid sulphonamides of the formula

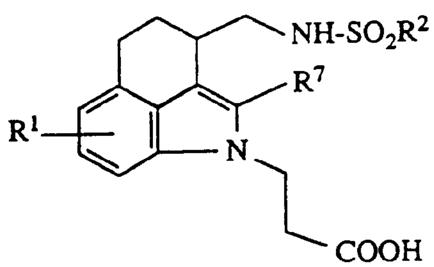

(I)

in which

$R^1$

– represents hydrogen, fluorine, chlorine, bromine, trifluoromethyl, carboxyl or $C_1$-$C_6$-alkoxycarbonyl,

or

– represents a group $-S(O)_nR^3$, $NR^4R^5$ or $-OR^6$,

in which

$R^3$ - denotes $C_1$-$C_6$-alkyl or phenyl,

$R^4$ and $R^5$ are identical or different and denote hydrogen, $C_1$-$C_6$-alkyl, phenyl or benzyl,

$R^6$ - denotes hydrogen, $C_1$-$C_6$-alkyl, phenyl, benzyl or trifluoromethyl,

and

n - denotes a number 0 or 2,

$R^1$ - represents $C_1$-$C_6$-alkyl, $C_2$-$C_6$-alkenyl, cyclopropyl, cyclopentyl or cyclohexyl, which is optionally substituted by carboxyl, $C_1$-$C_6$-alkoxy, fluorine, chlorine, bromine, hydroxyl, $C_1$-$C_6$-alkoxycarbonyl, $C_1$-$C_6$-alkylthio or cyano,

$R^2$ - represents phenyl or naphthyl, which is mono-substituted to trisubstituted by identical or different substituents from the series consisting of fluorine, chlorine, bromine, cyano, trifluoromethyl, trifluoromethoxy, trifluoromethylthio, $C_1$-$C_6$-alkyl, carboxyethyl, carboxyethyl, methoxycarbonylmethyl, ethoxycarbonylmethyl, methoxycarbonylethyl, $C_1$-$C_6$-alkoxy, $C_1$-$C_6$-alkylthio, hydroxyl, carboxyl, $C_1$-$C_6$-alkoxycarbonyl,phenyl,phenoxy,benzyloxy or benzylthio,

or by a group of the formula $-NR^4R^5$,

in which

$R^4$ and $R^5$ have the abovementioned meaning,

$R^7$ - represents hydrogen, $C_1$-$C_6$-alkyl, cyclopropyl, cyclopentyl or cyclohexyl,

and their salts,

characterised in that tetrahydro-1-benz-[c,d]-indole-sulphonamides of the formula

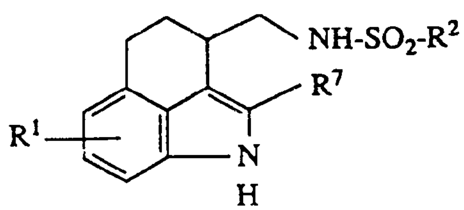

(IV)

in which

$R^1$, $R^2$ and $R^7$ have the abovementioned meaning, are reacted with acrylonitrile of the formula (V)

$$H_2C=CH\text{-}CN \qquad (V)$$

in the presence of an inert solvent and if appropriate in the presence of a base to give the compounds of the general formula (VI)

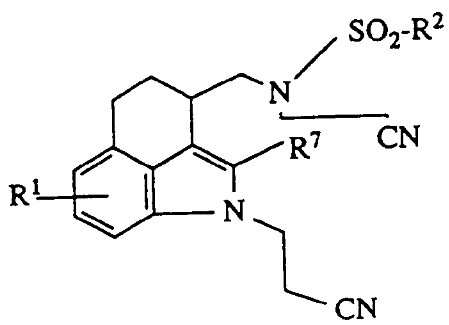

(VI)

in which

$R^1$, $R^2$ and $R^7$ have the abovementioned meaning,

and the N,N'-biscyanoethyl compounds (VI) are then hydrolysed.

2. Process according to Claim 1, characterised in that it is carried out in the temperature range from 0 to 150°C.

3. Process according to Claim 1, characterised in that the hydrolysis of the N,N'-bis-cyanoethyl compounds is carried out in a temperature range from 0°C to + 100°C.

## Revendications

### Revendications pour les Etats contractants suivants : AT, BE, CH, DE, FR, GB, GR, IT, LI, LU, NL, SE

1. Acides tétrahydro-1-benzo-[c,d]-indole-propioniques-sulfonamides de formule

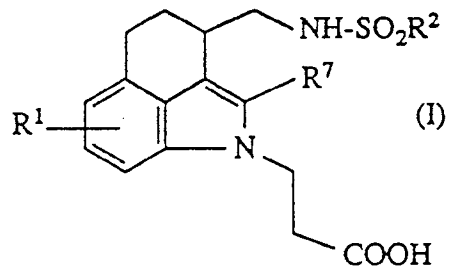

(I)

dans laquelle

$R^1$ représente l'hydrogène, le fluor, le chlore, le brome, un groupe trifluorométhyl, carboxy ou (alcoxy en $C_1$-$C_6$)-carbonyle, ou bien un groupe -S(O)$_n$R$^3$,NR$^4$R$^5$ ou -OR$^6$ dans lesquels

$R^3$ représente un groupe alkyle en $C_1$-$C_6$ ou phényle,

$R^4$ et $R^5$, ayant des significations identiques ou différentes, représentent chacun l'hydrogène, un groupe alkyle en $C_1$-$C_6$, phényle ou benzyle,

$R^6$ représente l'hydrogène, un groupe alkyle en $C_1$-$C_6$, phényle, benzyle ou trifluorométhyle, et n est égal à 0 ou 2,

ou bien

$R^1$ représente un groupe alkyle en $C_1$-$C_6$, alcényle en $C_2$-$C_6$, cyclopropyle, cyclopentyle ou cyclohexyle, qui peut le cas échéant être substitué par des groupes carboxy, alcoxy en $C_1$-$C_6$, le fluor, le chlore, le brome, des groupes hydroxy, (alcoxy en $C_1$-$C_6$)-carbonyle, alkylthio en $C_1$-$C_6$ ou cyano,

$R^2$ représente un groupe phényle ou naphtyle qui peut porter jusqu'à trois substituants identiques ou différents choisis parmi le fluor, le chlore, le brome, des groupes cyano, trifluorométhyle, trifluorométhoxy, trifluorométhylthio, alkyle en $C_1$-$C_6$, carboxyméthyle, carboxyéthyle, méthoxycarbonylméthyle, éthoxycarbonylméthyle, méthoxycarbonyléthyle, alcoxy en $C_1$-$C_6$, alkylthio en $C_1$-$C_6$, hydroxy, carboxy, (alcoxy en $C_1$-$C_6$)-carbonyle, phényle, phénoxy, benzyloxy, benzylthio,

ou un substituant de formule -NR$^4$R$^5$ dans laquelle

$R^4$ et $R^5$ ont les significations indiquées ci-dessus,

$R^7$ représente l'hydrogène, un groupe alkyle en $C_1$-$C_6$, cyclopropyle, cyclopentyle ou cyclohexyle, et leurs sels.

2. Acides tétrahydro-1-benzo-[c,d]-indole-propioniques-sulfonamides selon la revendication 1

dans lesquels

$R^1$ représente l'hydrogène, le fluor, le brome, un groupe trifluorométhyle, méthylthio, éthylthio, méthylsulfonyle, éthylsulfonyle, phénylthio, phénylsulfonyle, amino, diméthylamino, diéthylamino, ou bien un groupe -OR$^6$ dans lequel

$R^6$ représente l'hydrogène, un groupe méthyle, éthyle, propyle, phényle ou benzyle ou bien un groupe méthyle, éthyle, propyle ou isopropyle,

$R^2$ représente un groupe phényle ou naphtyle qui peut porter jusqu'à deux substituants identiques ou différents choisis parmi le fluor, le chlore, le brome, les groupes cyano, trifluorométhyle, trifluorométhoxy, méthyle, éthyle, propyle, méthoxy, éthoxy, propoxy, méthylthio, hydroxy, méthoxycarbonyle, éthoxycarbonyle, phényle, diméthylamino ou diéthylamino,

$R^7$ représente l'hydrogène ou un groupe méthyle,

et leurs sels.

3. Acides tétrahydro-1-benzo-[c,d]-indole-propioniques-sulfonamides selon les revendications 1 et 2, dans lesquels

$R^1$ représente l'hydrogène ou le fluor,

$R^2$ représente un groupe phényle ou naphtyle substitué par le fluor, le chlore, le brome, un groupe méthyle, méthoxy ou phényle,

$R^7$ représente un groupe méthyle,

et leurs sels.

4. Acides tétrahydro-1-benzo-[c,d]-indole-propioniques-sulfonamides selon les revendications 1 à 3, pour le traitement thérapeutique.

5. Procédé de préparation des acides tétrahydro-1-benzo-[c,d]-indole-propioniques-sulfonamides selon la

revendication 1, caractérisé en ce que l'on fait réagir des tétrahydro-1-benzo-[c,d]-indoles-sulfonamides de formule

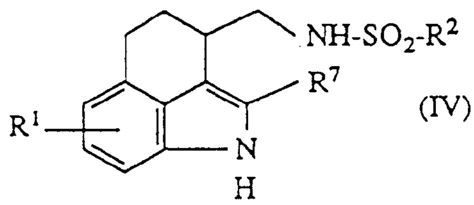

(IV)

dans laquelle $R^1$ , $R^2$ et $R^7$ ont les significations indiquées dans la revendication 1, avec l'acrylonitrile de formule V

$$H_2C = CH - CN \quad (V)$$

en présence d'un solvant inerte, éventuellement en présence d'une base, la réaction donnant des composés de formule générale VI

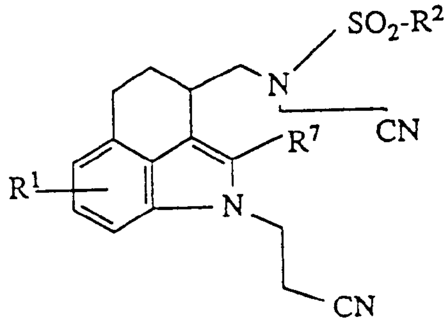

(VI)

dans laquelle $R^1$, $R^2$ et $R^7$ ont les significations indiquées ci-dessus, ces dérivés N,N'-bis-cyanéthyliques VI étant ensuite soumis à saponification.

6. Procédé selon la revendication 5, caractérisé en ce que l'on opère dans l'intervalle de température de 0 à 150°C.

7. Tétrahydro-1-benzo-[c,d]-indole-sulfonamides de formule

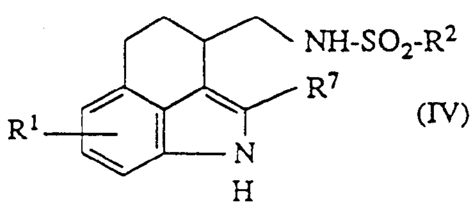

(IV)

dans laquelle
$R^1$ représente l'hydrogène, le fluor, le chlore, le brome, un groupe trifluorométhyle, carboxy ou (alcoxy en $C_1$-$C_6$)-carbonyle,
ou bien
un groupe $-S(O)_nR^3$,$NR^4R^5$ ou $-OR^6$ dans lesquels
$R^3$ représente un groupe alkyle en $C_1$-$C_6$ ou phényle,
$R^4$ et $R^5$, ayant des significations identiques ou différentes, représentent chacun l'hydrogène, un groupe alkyle en $C_1$-$C_6$, phényle ou benzyle,
$R^6$ représente l'hydrogène, un groupe alkyle en $C_1$-$C_6$, phényle, benzyle ou trifluorométhyle, et
n est égal à 0 ou 2,
ou bien
$R^1$ représente un groupe alkyle en $C_1$-$C_6$, alcényle en $C_2$-$C_6$, cyclopropyle, cyclopentyle ou cyclohexyle éventuellement substitué par des groupes carboxy, alcoxy en $C_1$-$C_6$, le fluor, le chlore, le brome, des grou-

pes hydroxy, (alcoxy en $C_1$-$C_6$)-carbonyle, alkylthio en $C_1$-$C_6$ ou cyano,
$R^2$ représente un groupe phényle ou naphtyle qui peut porter jusqu'à trois substituants identiques ou différents choisis parmi le fluor, le chlore, le brome, des groupes cyano, triflurorométhyle, trifluorométhoxy, trifluorométhylthio, alkyle en $C_1$-$C_6$, carboxyméthyle, carboxyéthyle, méthoxycarbonylméthyle, éthoxycarbonylméthyle, méthoxycarbonyléthyle, alcoxy en $C_1$-$C_6$, alkylthio en $C_1$-$C_6$, hydroxy, carboxy, (alcoxy en $C_1$-$C_6$)-carbonyle, phényle, phénoxy, benzyloxy, benzylthio,
ou un substituant de formule $-NR^4R^5$ dans lequel
$R^4$ et $R^5$ ont les significations indiquées ci-dessus,
$R^7$ représente l'hydrogène, un groupe alkyle en $C_1$-$C_6$, cyclopropyle, cyclopentyle ou cyclohexyle,
et leurs sels.

8. Procédé de préparation des tétrahydro-1-benzo-[c,d]-indole-sulfonamides selon la revendication 7, caractérisé en ce que l'on fait réagir des indoles de formule

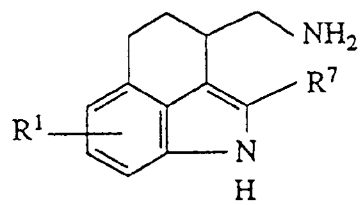

(VII)

dans laquelle $R^1$ et $R^7$ ont les significations indiquées dans la revendication 7, avec des halogénures d'acides sulfoniques de formule générale VIII

$$X\text{-}SO_2\text{-}R^2 \qquad (VIII)$$

dans laquelle $R^2$ a les significations indiquées dans la revendication 7, et X représente un halogène, dans des solvants inertes, éventuellement en présence d'une base.

9. Médicaments contenant des acides tétrahydro-1-benzo-[c,d]-indole-propioniques-sulfonamides selon les revendications 1 à 3.

10. Médicament selon la revendication 9, contenant de 0,5 à 90 % en poids d'acides tétrahydra-1-benzo-[c,d]-indole-propioniques-sulfonamides par rapport à la composition.

11. Utilisation des acides tétrahydro-1-benzo-[c,d]-indole-propioniques-sulfonamides selon les revendications 1 à 3 pour la préparation de médicaments.

**Revendications pour l'Etat contractant suivant : ES**

1. Procédé de préparation d'acides tétrahydro-1-benzo-[c,d]-indole-propioniques-sulfonamides de formule

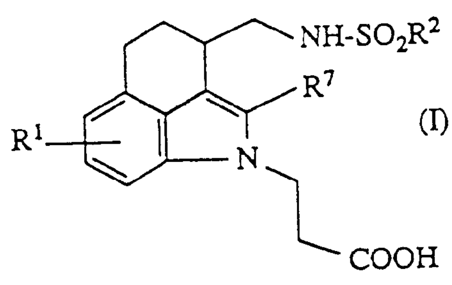

(I)

dans laquelle
$R^1$ représente l'hydrogène, le fluor, le chlore, le brome, un groupe trifluorométhyl, carboxy ou (alcoxy en $C_1$-$C_6$)-carbonyle, ou bien un groupe $-S(O)_nR^3$,$NR^4R^5$ ou $-OR^6$ dans lesquels
$R^3$ représente un groupe alkyle en $C_1$-$C_6$ ou phényle,
$R^4$ et $R^5$, ayant des significations identiques ou différentes, représentent chacun l'hydrogène, un groupe alkyle en $C_1$-$C_6$, phényle ou benzyle,
$R^6$ représente l'hydrogène, un groupe alkyle en $C_1$-$C_6$, phényle, benzyle ou trifluorométhyle, et

n est égal à 0 ou 2,
ou bien

$R^1$ représente un groupe alkyle en $C_1$-$C_6$, alcényle en $C_2$-$C_6$, cyclopropyle, cyclopentyle ou cyclohexyle, qui peut le cas échéant être substitué par des groupes carboxy, alcoxy en $C_1$-$C_6$, le fluor, le chlore, le brome, des groupes hydroxy, (alcoxy en $C_1$-$C_6$)-carbonyle, alkylthio en $C_1$-$C_6$ ou cyano,

$R^2$ représente un groupe phényle ou naphtyle qui peut porter jusqu'à trois substituants identiques ou différents choisis parmi le fluor, le chlore, le brome, des groupes cyano, trifluorométhyle, trifluorométhoxy, trifluorométhylthio, alkyle en $C_1$-$C_6$, carboxyméthyle, carboxyéthyle, méthoxycarbonylméthyle, éthoxycarbonylméthyle, méthoxycarbonyléthyle, alcoxy en $C_1$-$C_6$, alkylthio en $C_1$-$C_6$, hydroxy, carboxy, (alcoxy en $C_1$-$C_6$)-carbonyle, phényle, phénoxy, benzyloxy, benzylthio,

ou un substituant de formule $-NR^4R^5$ dans laquelle

$R^4$ et $R^5$ ont les significations indiquées ci-dessus,

$R^7$ représente l'hydrogène, un groupe alkyle en $C_1$-$C_6$, cyclopropyle, cyclopentyle ou cyclohexyle,

et leurs sels,

caractérisé en ce que l'on fait réagir des tétrahydro-1-benzo-[c,d]-indole-sulfonamides de formule

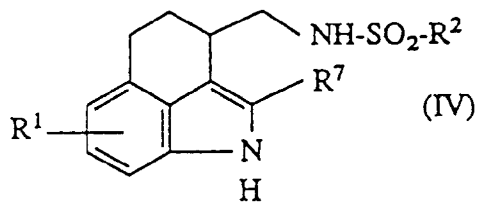

(IV)

dans laquelle $R^1$ , $R^2$ et $R^7$ ont les significations indiquées ci-dessus, avec l'acrylonitrile de formule V

$$H_2C = CH - CN \quad (V)$$

en présence d'un solvant inerte, éventuellement en présence d'une base, la réaction donnant des composés de formule générale VI

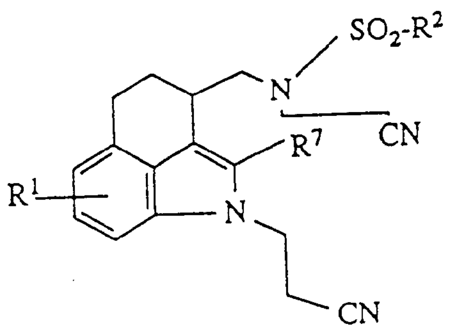

(VI)

dans laquelle $R^1$, $R^2$ et $R^7$ ont les significations indiquées ci-dessus,

ces dérivés N,N'-bis-cyanéthyliques VI étant ensuite soumis à saponification.

2. Procédé selon la revendication 1, caractérisé en ce que l'on opère dans l'intervalle de température de 0 à 150°C.

3. Procédé selon la revendication 1, caractérisé en ce que la saponification des dérivés N,N'-bis-cyanéthyliques est réalisée dans l'intervalle de température de 0 à +100°C.